(19) Europäisches Patentamt
European Patent Office
Office européen des brevets

(11) Publication number: 0 321 490 B1

# (12) EUROPEAN PATENT SPECIFICATION

(45) Date of publication of patent specification: **22.06.94**

(51) Int. Cl.[5]: **A61K 31/44**

(21) Application number: **87905839.4**

(22) Date of filing: **17.08.87**

(86) International application number:
**PCT/US87/02014**

(87) International publication number:
**WO 88/01169 (25.02.88 88/05)**

## (54) INHIBITION OF INTERLEUKIN-1 PRODUCTION BY MONOCYTES AND/OR MACROPHAGES.

(30) Priority: **19.08.86 US 897909**
**17.06.87 US 63550**

(43) Date of publication of application:
**28.06.89 Bulletin 89/26**

(45) Publication of the grant of the patent:
**22.06.94 Bulletin 94/25**

(84) Designated Contracting States:
**AT BE CH DE FR GB IT LI LU NL SE**

(56) References cited:
**GB-A- 2 039 882**
**US-A- 4 064 260**
**US-A- 4 110 460**
**US-A- 4 153 706**
**US-A- 4 175 127**
**US-A- 4 186 205**
**US-A- 4 263 311**

**J. MEDICINAL CHEMISTRY, vol. 28, 1985; P.E. BENDER et al., pp. 1169-1177&NUM;**

**J. MEDICINAL CHEMISTRY, vol. 27, 1984; I. LANTOS et al., pp. 72-75&NUM;**

(73) Proprietor: **SMITHKLINE BEECHAM CORPORATION**
**One Franklin Plaza**
**P.O. Box 7929**
**Philadelphia Pennsylvania 19103(US)**

(72) Inventor: **BENDER, Paul, Elliot**
**504 Lilac lane**
**Cherry Hill, NJ 08003(US)**
Inventor: **GRISWOLD, Don, Edgar**
**205 Lower Valley Road**
**North Wales, PA 19454(US)**
Inventor: **HANNA, Nabil**
**507 Kent Place**
**Berwyn, PA 19312(US)**
Inventor: **LEE, John, C.**
**245 Gulph Hills Road**
**Radnor, PA 19087(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 321 490 B1

**ARTHRITIS AND RHEUMATISM, vol. 26, no. 8, August 1983; D.D. WOOD et al., pp. 975-983&NUM;**

**J. LEUKOCYTE BIOLOGY, vol. 37, 1985; T. IKEJIMA et al., pp. 714-715&NUM;**

**J. LEUKOCYTE BIOLOGY, vol. 37, 1985; G.S. HABICHT et al., p. 709&NUM;**

(74) Representative: **Thompson, Clive Beresford et al**
**SmithKline Beecham plc**
**Corporate Patents**
**Mundells**
**Welwyn Garden City, Herts AL7 2EY (GB)**

## Description

This invention relates to a method of inhibiting the production of interleukin-1 (IL-1) by monocytes and/or macrophages in a human in need thereof which comprises administering to such human an effective, IL-1 production inhibiting amount of a compound of the formula (I)

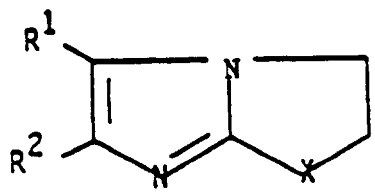

Formula (I)

wherein:

One of $R^1$ and $R^2$ must be 4-pyridyl and the other is selected from 4-pyridyl or monosubstituted phenyl wherein said substituent is selected from halo or $C_{1-4}$ alkoxy;

X is $CH_2$, $CH_2CH_2$ or S(0)n; and

n is 0, 1 or 2;

or a pharmaceutically acceptable salt thereof.

The preparation of all compounds of Formula (I) and pharmaceutically acceptable salts thereof is disclosed in Bender et al., U.S. Patent Application Serial Number 856,875 filed April 28, 1986 and Bender et al., U.S. Patent Application Serial Number 856,928, filed April 28, 1986, the chemical synthetic disclosures of both of which are hereby briefly described:

As used herein, the term "Formula (Z)" will refer to compounds represented by the structure:

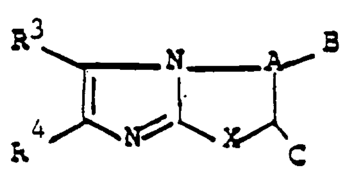

Formula (Z)

wherein:

A is $CH_2$ or $CH_2CH_2$;

B and C are independently selected from H, methyl, ethyl or dimethyl;

when A is $CH_2CH_2$, B is a substituent on either or both carbon atoms;

X is $CH_2$ or $S(O)_n$ and n is 0, 1, or 2;

$R^3$ and $R^4$ are independently selected from

(a) pyridyl,

(b) phenyl or monosubstituted phenyl wherein said substituent is selected from $C_{1-3}$ dialkylamino, $C_{1-3}$ alkylamino, N-(azacyclo $C_{5-6}$ alkyl), cyano, 2,2,2-trihaloethoxy, prop-2-ene-1-oxy, N-($C_{1-3}$ alkyl)-($C_{1-3}$ alkanamido), $C_{1-3}$ alkanamido, amino, hydroxy, $C_{1-3}$ alkylthio, $C_{1-4}$ alkyl, halo, $CF_3$, $C_{1-3}$alkoxy, $C_{1-3}$ alkylsulfinyl, or $C_{1-3}$ alkylsulfonyl;

(c) disubstituted phenyl wherein said substituents are independently selected from $C_{1-4}$ alkyl, $C_{1-3}$ alkoxy, hydroxy, nitro, amino, halo, $C_{1-3}$ alkylamino, $C_{1-3}$ dialkylamino, N-(azacyclo $C_{5-6}$ alkyl), 2,2,2-trihaloethoxy, prop-2-ene-1-oxy, or N-($C_{1-3}$ alkyl)-($C_{1-3}$alkanamido), or the disubstituents together form a methylenedioxy group; or

(d) 3,4,5-trimethoxyphenyl;

provided that:

(1) when $R^3$ is cyanophenyl, $R^2$ must be either cyanophenyl or 4-pyridyl;
(2) when one of $R^2$ or $R^3$ is hydroxy, the other must be pyridyl, halo, $CF_3$, $C_{1-4}$ alkyl, $C_{1-3}$ alkylamino, $C_{1-3}$ dialkylamino, or N-(azacyclo $C_{3-6}$ alkyl;
(3) when X is $CH_2$, both $R^2$ and $R^3$ can be amino-substituted phenyl;

or a pharmaceutically acceptable salt thereof.

All the compounds of Formula (Z) can be prepared by the following synthetic routes 1 or 2.

All the compounds of Formula (I) are within the scope of Formula Z.

## SYNTHETIC ROUTE 1

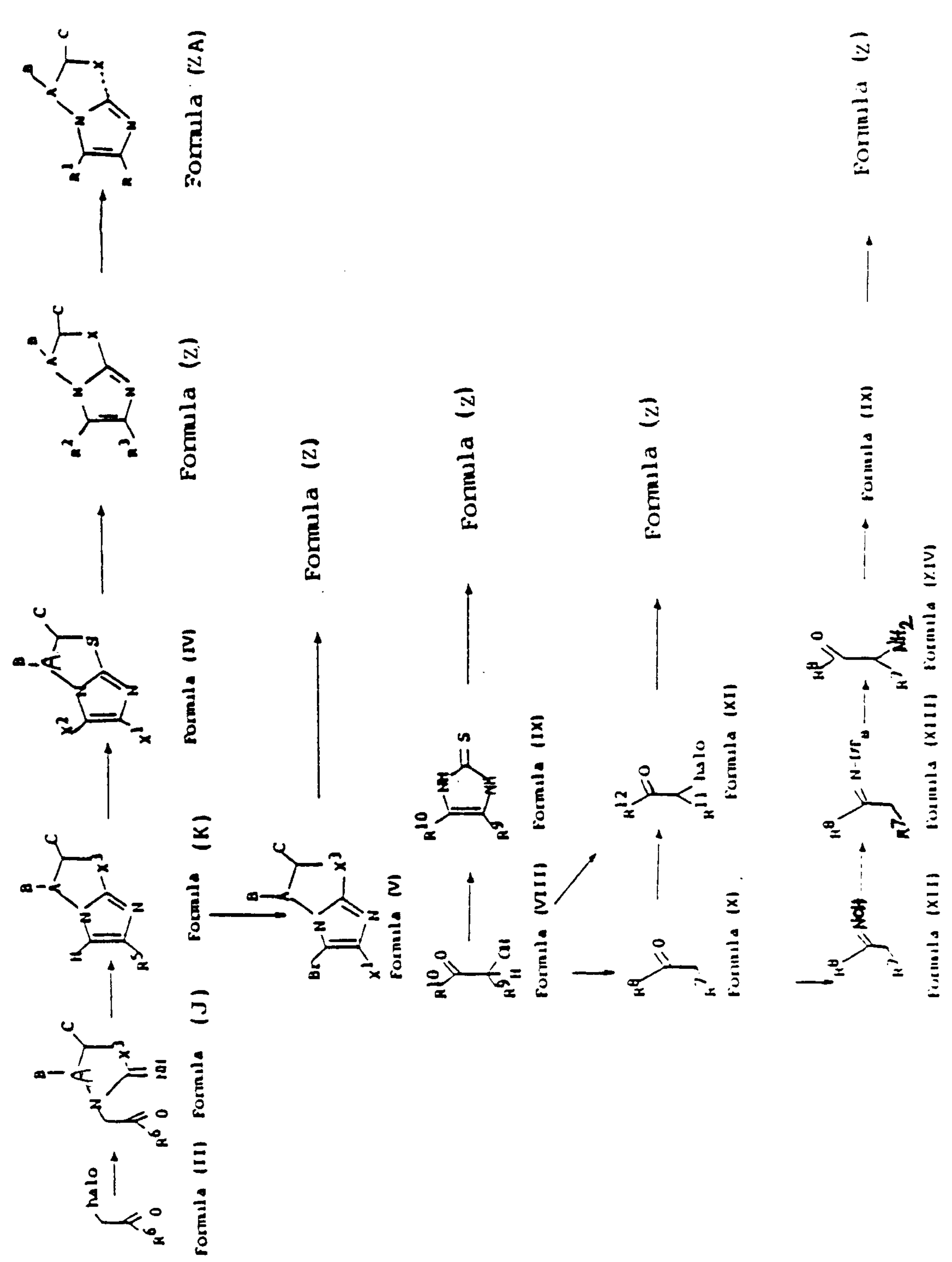

## SYNTHETIC ROUTE 2

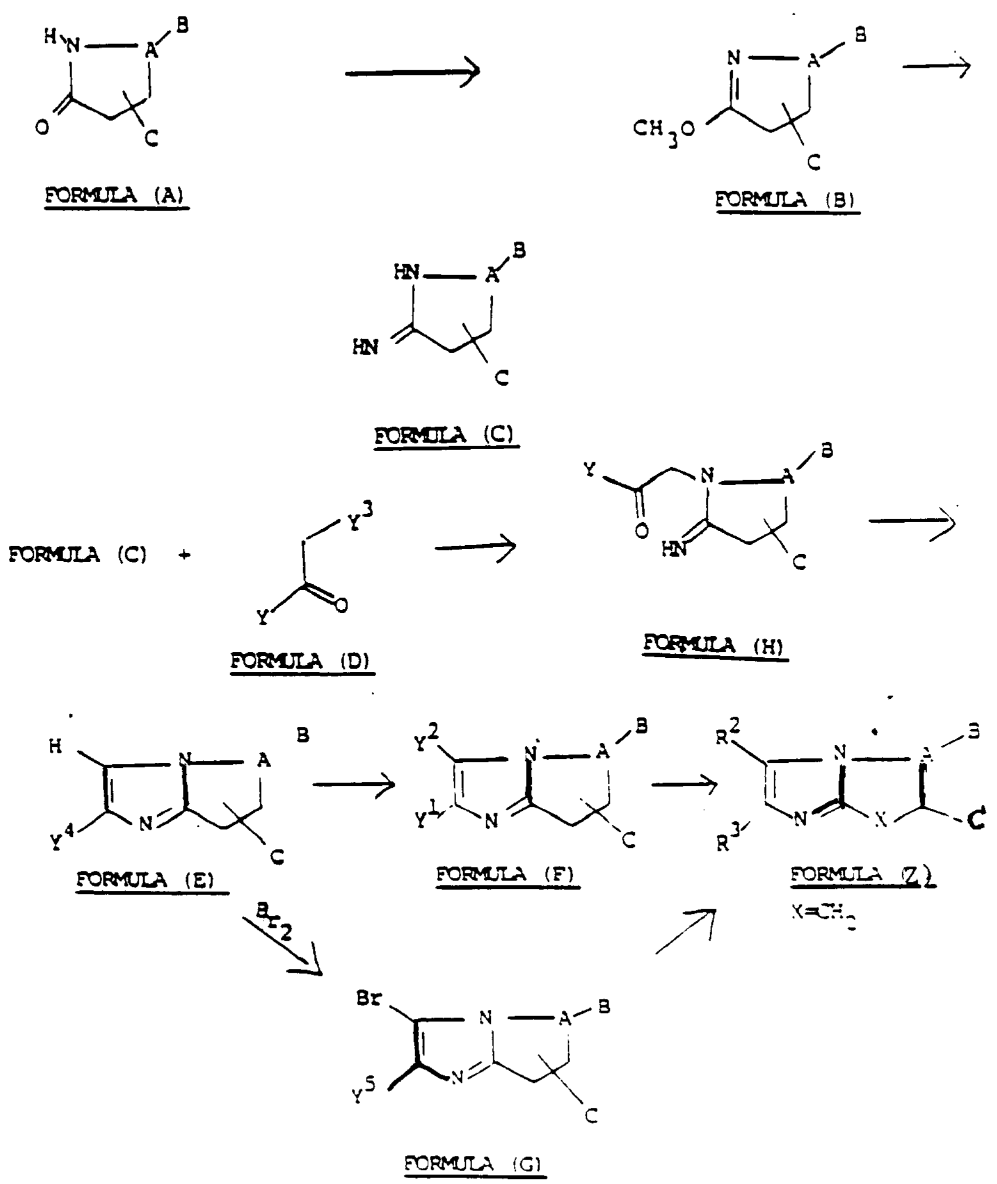


FORMULA (A)
FORMULA (B)
FORMULA (C)
FORMULA (C) +
FORMULA (D)
FORMULA (H)
FORMULA (E)
FORMULA (F)
FORMULA (Z)
FORMULA (G)

It will be apparent to one of skill in the art that all the compounds of Formula (ZA), Formula (IB) and Formula (IC) are encompassed by the scope of Formula (Z).

The required compounds necessary to produce the compounds of Formula (Z) in accordance with synthetic route 1, i.e., the compounds of Formulas (II), (J), (K), (IV), (V), (VIII), (IX), (X), (XI), (XII), (XIII), (XIV) and (Z), can be obtained from commercial sources or can be prepared by techniques described herein.

The compounds of Formula (Z) can be prepared according to synthetic route 1 by means of the following reactions.

The compounds of Formula (K) are prepared in two steps from the corresponding Formula (II) and Formula (J) compounds. Formula (J) compounds are prepared by treatment of the appropriately substituted Formula (II) phenacyl halide with the correspondingly substituted 2-amino-3,4-dihydrothiazole or 2-amino-5,6-dihydro-4H-(1,3)thiazinein a chlorinated hydrocarbon or preferably an alcoholic solvent. It has been

found that a polar solvent is also effective. The hydrohalide salt of the Formula (J) compound which can be isolated as a precipitate or oil on addition of a nonpolar cosolvent such as ether or hexane, is refluxed in water or aqueous alcohol until cyclodehydration is complete. Neutralization with aqueous base affords the corresponding Formula (K) compound. The substituted Formula (II) compounds are prepared from the corresponding acetophenones by treatment with bromine or alternatively prepared by acylting the corresponding mono- or disubstituted benzene by Friedel Crafts reaction with 2-chloroacetyl chloride and $AlCl_3$.

The Formula (K) 6-arylimidazo[2,1-b]thiazole or thiazine analog is reacted with an excess of both the pyridine and the reactive acyl ester in a 2:1 ratio in an organic solvent in which the reactants are soluble and inert to prepare a compound of Formula (IV). One skilled in the art will recognize that the pyridine and acyl ester react to form the acylpyridinium reagent in situ. Optionally, the acylpyridinium reagent can be prepared separately in a solvent and then added to the solution of Formula (K) compound. Suitable solvents include methylene chloride, ethylene chloride, chloroform, carbon tetrachloride, tetrahydrofuran, ethyl ether, dioxane, toluene, or excess pyridine.

The reaction mixture is cooled during mixing of the reactants, preferably to a temperature between 5-20 °C, by use of an ice/water bath. The mixture is then allowed to stand briefly at ambient temperature, followed by refluxing until the reaction is complete. The reaction mixture is continually assayed using high pressure liquid chromatography (HPLC) or thin layer chromatography (TLC) on aliquots to ascertain if unreacted compound is present. If so, additional acyl pyridinium salt is introduced. Following the reaction, the resultant Formula (IV) compounds can be recovered from the reaction mixture and isolated by standard techniques.

The compound of Formula (K) in a nonpolar organic solvent is treated with bromine at room temperature. The resulting hydrobromide salt of Formula (V) is treated with aqueous base to afford the Formula (V) compound.

The Grignard reagent derived from a compound of Formula (V) is prepared by reacting a compound of Formula (V) with at least an equimolar amount of a $C_{1-5}$ alkyllithium reagent at a temperature of from about -80 °C to about -30 °C. Following the lithium-halogen exchange, from which the organolithium compound results, magnesium halide etherate is added to the mixture in excess and reacted with the organolithium compound during which the reaction temperature can be permitted to rise to a temperature of about 0 °C, although a reaction temperature below about -10 °C is preferred.

Compounds of Formula (IV) can also be prepared by reacting the Grignard reagent derived from a Formula (V) compound with an excess of both pyridine and the selected reactive acyl ester in a solvent such as ethyl ether, or preferably, tetrahydrofuran. The Grignard reagent can be combined with the pyridine and the acyl ester in sequence, or the pyridinium salt can be prepared separately and then added to the solution of Grignard reagent. Preferably, the reaction is conducted by the addition of 2-6 moles of pyridine to each mole of the Grignard reagent, followed by addition of at least an equimolar amount of the acyl compound.

This reaction is conducted at a temperature of -10 °C or below. A minimum of about 5 mole percent of cuprous iodide (CuI), based on the molar quantity of pyridine present, is introduced to the reaction mixture to insure the ultimate addition of the Grignard reagent at the 4-position of the N-acyl-1,4-dihydropyridine compound.

The reaction mixture can be permitted to warm to a temperature of about 20 °C, but preferably no higher than about 15 °C. Aliquots of the reaction mixture are removed at intervals and assayed using HPLC or TLC to determine the presence of unreacted imidazo[2,1-b]thiazole or thiazine. The conditions of the reaction are otherwise standard for those pertaining to Grignard synthesis in general. Recovery and isolation can be effected by standard techniques.

The Formula (K) starting materials for the Formula (IV) compounds in which A is $CH_2CH_2$ (thiazine) can be prepared in an analogous manner by heating the corresponding 2-amino-5,6-dihydro-4H-1,3-thiazine with a 4-substituted bromo-acetophenone of Formula (II) in a non-polar solvent such as benzene or chloroform, to form an intermediate which is then refluxed in water. It has also been found that a polar solvent can be used as well, and that the intermediate of Formula (J) may or may not be isolated prior to the refluxing step. The amino-thiazine starting material can be prepared by treating the appropriate 3-bromopropylamine with t-butyl isothiocyanate, followed by reflux with hydrobromic or hydrochloric acid.

Compounds of Formula (Z) wherein X is S; A is $CH_2$ or $CH_2CH_2$; B and C are independently selected from H, methyl, ethyl, or gem-dimethyl; and $R^3$ and $R^2$ are independently mono-, di- or tri-substituted phenyl, are prepared from either (a) the corresponding Formula (IX) compounds wherein $R^9$ or $R^{10}$ are the same as $R^a$ or $R^b$ in Formula (IB); or (b) the corresponding Formula (XI) compounds wherein $R^{11}$ or $R^{12}$ are the same as $R^a$ or $R^b$ in Formula (IB).

In accordance with synthetic route 1, in the first procedure, the Formula (IX) compound is treated with one equivalent of the corresponding 1,2-dihaloethane or 1,3-dihalopropane and potassium carbonate. The reactants are refluxed in N,N-dimethylformamide for about 3 hours, cooled, water is added, and the pH is adjusted to 11 with a base such as 10% aqueous sodium hydroxide. The precipitate is washed with water and dissolved in a solvent such as methylene chloride. The organic solution is washed with water, dried, and treated with charcoal. The solvent is removed and the crude product recrystallized or, alternatively, it is chromatographed and the solvent evaporated in vacuo.

The requisite Formula (IX) compounds wherein $R^9$ and $R^{10}$ are independently selected from (a) phenyl or monosubstituted phenyl where said substituent is selected from $C_{1-3}$ dialkylamino, N-(azacyclo $C_{5-6}$ alkyl), $C_{1-3}$ alkoxy, 2,2,2-trihaloethoxy, prop-2-ene-1-oxy, $C_{1-4}$ alkyl, $C_{1-3}$ alkylthio, $C_{1-3}$ alkanamido, N-($C_{1-3}$ alkyl)-($C_{1-3}$ alkanamido), halo, or $CF_3$, (b) disubstituted phenyl wherein said substitutents are independently selected from $C_{1-4}$ alkyl or $C_{1-3}$ alkoxy or the disubstituents together form a methylenedioxy group, or (c) 3,4,5-trimethoxyphenyl are prepared by refluxing the corresponding Formula (VIII) compound with thiourea in dimethylformamide. The Formula (VIII) compounds are prepared from the condensation of two molecules of the corresponding aryl carboxaldehydes catalyzed by cyanide, usually in refluxing aqueous ethanolic solution (benzoin condensation).

Alternatively, compounds of Formula (IX) may be prepared from the corresponding ethanones of Formula (X) by reaction with hydroxylamine to afford the oximes of Formula (XII). Subsequent treatment of the Formula (XII) compounds with tosyl chloride and pyridine gives the oxime tosylates of Formula (XIII). Reaction of the Formula (XIII) compounds with strong base affords the 2-amino-ethan-1-ones of Formula (XIV). These compounds are treated with hydrochloric acid, and the resulting Formula (XIV) hydrochloride salt is refluxed in an aqueous solution of sodium thiocyanate to afford conversion of the Formula (XIV) compound into the diaryl-2-mercaptoimidazole Formula (IX) compound which can be filtered from the reaction mixture and recrystallised in alcohol or dimethylformamide-water. The required ethanones of Formula (X) wherein $R^7$ and $R^8$ are independently selected from (a) phenyl or monosubstituted phenyl where said substitutent is selected from $C_{1-3}$ alkoxy, 2,2,2-trihaloethoxy, prop-2-ene-1-oxy, $C_{1-4}$ alkyl, $C_{1-3}$ alkylthio, $C_{1-3}$ alkanamido, halo, of $CF_3$ (b) disubstituted phenyl wherein said substituents are independently selected from $C_{1-4}$ alkyl or $C_{1-3}$ alkoxy or the disubstituents together form a methylenedioxy group (c) 3,4,5-trimethoxyphenyl or (d) $R^7$ and $R^8$ are both CN, are obtained by (a) Friedel-Crafts acylation by a substituted phenylacetyl chloride on the corresponding benzene, (b) Curtius rearrangement of the substituted stilbenecarbonyl azides derived from Perkin condensation of a substituted benzaldehyde with the substituted phenylacetic acid ester, (c) reduction of the corresponding benzoin with zinc or tin in glacial acetic acid by boiling for 4-5 hours, and (d) Claisen condensation of a substituted phenylacetonitrile with a substituted aryl carboxylic acid ester.

In the second procedure of synthetic route 1 for the preparation of Formula (Z) compounds wherein X is S; A is $CH_2$ or $CH_2CH_2$; B and C are independently selected from H, methyl, ethyl, or gem-dimethyl; and $R^3$ and $R^2$ are independently mono-, di- or tri-substituted phenyl, one equivalent of a 2-halo-ethan-1-one of Formula (XI) wherein $R^{11}$ and $R^{12}$ are independently selected from (a) phenyl or monosubstituted phenyl and said substituent is selected from $C_{1-4}$ alkyl, halo (preferably chloro or bromo), $CF_3$, $C_{1-3}$ alkoxy, $C_{1-3}$ alkylthio, 2,2,2-trihaloethoxy, prop-2-ene-1-oxy, $C_{1-3}$ alkanamido, N-($C_{1-3}$ alkyl)-($C_{1-3}$ alkanamido), or (b) disubstituted phenyl wherein said substituents are independently selected from $C_{1-4}$ alkyl or $C_{1-3}$ alkoxy or the disubstituents together form a methylenedioxy group, is treated wtih two to five equivalents of the correspondingly substituted 2-amino-thiazoline or 2-amino-5,6-dihydro-4H-[1,3]thiazine and potassium carbonate in a polar solvent such as dry acetonitrile at room temperature for 18 hours to 5 days. The product is then purified, for example the solvent is evaporated and the residue is dissolved in methylene chloride, is washed with 5% aqueous sodium carbonate and the organic phase concentrated in vacuo and recrystallized or chromatographed to yield the desired Formula (Z) compound. The Formula (XI) compounds where halo is Br are prepared preferably by bromination of the corresponding Formula (X) compounds. Alternately the alpha-chloro Formula (XI) compounds are prepared from the corresponding Formula (VIII) compounds by heating of 4 g of the benzoin with 4 ml of thionyl chloride.

Alternatively, when X is $CH_2$ or S, one equivalent of 2-haloethanone of Formula (XI) is treated with one equivalent of the corresponding substituted 2-amino-thiazoline or 2-amino-4,5-dihydro-[1,3]thiazine (where X is S) or 2-imino-pyrrolidine or 2-iminopiperidine (where X is $CH_2$) in a nonpolar solvent, such as chloroform or toluene, at room temperature for about 4 to 24 hours. The solvent is evaporated in vacuo and the residue refluxed in a polar solvent such as water or aqueous alcohol for up to about 15 hours. The solution is treated with 5% aqueous sodium carbonate and extracted with methylene chloride. The organic phase is concentrated in vacuo and recrystallized or chromatographed to yield the desired Formula (Z) product.

Compounds of Formula (Z) wherein X is S; A is $CH_2$ or $CH_2CH_2$; B and C are independently selected from H, methly, ethyl, or gem-dimethyl; and one of $R^2$ or $R^3$ is pyridyl and the other is mono-, di-, or trisubstituted phenyl are prepared from the corresponding Formula (IX) compounds by alkylation with the appropriate $C_{2-3}$ dihaloalkane and one equivalent of sodium hydride in dimethylformamide followed by addition of potassium carbonate, subsequent cyclization upon heating, and precipitation by addition of ice water. The resulting two isomeric 6-aryl-5-pyridyl and 5-aryl-6-pyridyl Formula (Z) compounds are separated chromatographically. The Formula (IX) compounds are prepared from either the corresponding (a) Formula (VIII) 2-hydroxyethanones or (b) Formula (XIV) 2-aminoethanones. Formula (IX) compounds where at least one of $R^9$ or $R^{10}$ is pyridyl and the other is selected from (a) phenyl or monosubstituted phenyl where said substituent is selected from $C_{1-3}$ alkoxy, 2,2,2-trihaloethoxy, prop-2-ene-1-oxy, $C_{1-4}$alkyl, $C_{1-3}$ alkylthio, $C_{1-3}$ alkanamido, halo, or $CF_3$, (b) disubstituted phenyl wherein said substituents are independently selected from $C_{1-4}$ alkyl or $C_{1-3}$ alkoxy or the disubstituents together form a methylenedioxy group, (c) 3,4,5-trimethoxyphenyl, or (d) pyridyl are prepared from the corresponding Formula (VIII) compounds by the same procedure as described above for the substituted diphenyl Formula (IX) compounds. Formula (VIII) compounds where one or $R^9$ or $R^{10}$ is 4-pyridyl are prepared by treatment of 4-pyridine carboxaldehyde cyanohydrin benzoate and a substituted benzaldehyde in t-butanol with sodium or potassium hydride.

Alternatively, pyridyl-containing Formula (IX) compounds may be prepared from the corresponding Formula (X) ethanones in 4 steps by successive conversion of the Formula (X) compounds to the corresponding Formula (XII) Formula (XIII), and Formula (XIV) compounds by the method previously described. The requisite Formula (X) ethanones wherein at least one of $R^7$ and $R^8$ is pyridyl and the other is independently selected from (a) phenyl or monosubstituted phenyl where said substituent is selected from $C_{1-3}$ alkoxy, 2,2,2-trihaloethoxy, prop-2-ene-1-oxy, $C_{1-4}$ alkyl, $C_{1-3}$ alkylthio, $C_{1-3}$-alkanamido, halo, or $CF_3$, (b) disubstituted phenyl wherein said substituents are independently selected from $C_{1-4}$ alkyl or $C_{1-3}$ alkoxy or the disubstituents together form a methylenedioxy group, or (c) 3,4,5-trimethoxyphenyl, are preferably prepared by Claisen condensation of a substituted phenylacetonitrile with a 2-, 3-, or 4-picolinic acid ester or alternatively by reaction of the picolyl sodium or lithium and the appropriately substituted benzoic acid ester.

The Formula (Z) compounds where both $R^2$ and $R^3$ are pyridyl and X is S are prepared in two steps from the corresponding Formula (VIII) 2-hydroxy-ethan-1-ones and the corresponding Formula (IX) compounds by the methods described above for the corrsponding Formula (VIII), Formula (IX) and Formula (Z) compounds where the aryl groups are both substituted phenyl. The precursor dipyridyl Formula (VIII) compounds are prepared by the benzoin condensation as described above for the corresponding diphenyl Formula (VIII) compounds with the exception that thiourea must be added in the condensation of 4-pyridine carboxaldehyde.

Compounds of Formula (Z) where $R^2$ is 4-pyridyl and $R^3$ is substituted phenyl are preferably prepared in two steps from their corresponding Formula (K) and Formula (IV) compounds. The Formula (IV) compounds wherein A is $CH_2$ or $CH_2CH_2$, B and C are independently selected from H, methyl, ethyl, or dimethyl; $X^2$ is N-Z-carbonyl-1, 4-dihydro-4-pyridyl, Z is $C_{1-8}$ alkyl, $C_{1-8}$ alkoxy, phenyl, phenoxy, benzyl or benzyloxy, and $X^1$ is (a) phenyl, or monosubstituted phenyl and said substituent is selected from $C_{1-3}$ alkoxy, halo, $CF_3$, $C_{1-3}$ alkylthio, $C_{1-4}$ alkyl, N-(azacyclo $C_{5-6}$ alkyl), N-($C_{1-3}$ alkyl)-($C_{1-3}$ alkanamido), $C_{1-3}$ dialkylamino, cyano, 2,2,2-trihaloethoxy prop-2-ene-1-oxy or disubstituted phenyl wherein said substituents are independently selected from $C_{1-4}$ alkyl or $C_{1-3}$ alkoxy or the disubstituents together form a methylenedioxy group, are prepared from the corresponding Formula (K) compounds. In the first step, the Formula (K) compound is treated at 5-25 °C with pyridine and an acyl halide such as an alkyl chloroformate (preferably ethyl chloroformate) or an arylcarbonyl halide such as benzoyl chloride in a solvent in which the reactants are soluble and inert, such as methylene chloride, to form the corresponding Formula (IV) compound. In the second step, the Formula (IV) compound, an N-acyldihydropyridine product, is deacylated and aromatized either with a mild oxidizing agent such as sulfur in refluxing decalin, tetralin or p-cymene or preferably with oxygen and excess potassium t-butoxide in t-butanol, to afford the compounds of Formula (Z).

The 2,2,2-trihaloethoxyphenyl and prop-2-ene-1-oxyphenyl substituted Formula (Z), Formula (K), and Formula (V) compounds are prepared by alkylation of the corresponding Formula (Z), Formula (K), and Formula (V) hydroxyphenyl compounds with a 2,2,2-trihaloalkylester of trifluoromethane sulfonic acid and 2-propenyl bromide respectively as follows. One equivalent of the hydroxyphenyl compound is added to sodium hydride in tetrahydrofuran under nitrogen below 0 °C. After 1/2 hour three equivalents of the ester of the trifluoromethane sulfonic acid is added dropwise. The suspension is poured into ice water and extracted into methylene chloride followed by washing, drying, and evaporation of the solvent. To one equivalent of

the hydroxyphenyl compound is added about one equivalent of the 2-propenyl bromide in dimethylformamide. Sodium hydride is added maintaining the temperature below 55°C. A second portion of the bromide is added and the reaction is heated to 60-75°C for 1 hour. The reaction mixture is cooled, is added to water, and the pH is adjusted to 11 with 10% sodium hydroxide. It is then extracted into methylene chloride and purified.

The hydroxyphenyl Formula (Z), Formula (K), and Formula (V) compounds are prepared by treatment of the corresponding methoxyphenyl compounds with HBr in refluxing acetic acid or alternatively with $BBr_3$ in methylene chloride.

$C_{1-3}$ alkanamido and N-($C_{1-3}$ alkyl-)($C_{1-3}$ alkanamido) phenyl substituted acetophenones, and in some cases the Formula (K), and Formula (Z) compounds, are prepared by acylation of the corresponding amino and $C_{1-3}$ alkylamino compounds with the alkanoic acid anhydride or chloride in pyridine. Another alternative preparation of the ($C_{1-3}$ alkyl)-($C_{1-3}$ alkanamido) phenyl carboxaldehydes, substituted Formula (K) and Formula (Z) compounds employs the alkylation of the corresponding ($C_{1-3}$ alkanamido) phenyl substituted carboxaldehydes, Formula (K) and Formula (Z) compounds with sodium hydride and a $C_{1-3}$ alkyl bromide or iodide in dimethylformamide.

Aminophenyl substituted Formula (K) and Formula (Z) compounds are prepared by hydrolysis of the corresponding $C_{1-3}$ alkanamido compounds in refluxing 6N mineral acid.

N-($C_{1-3}$ alkylamino) phenyl substituted Formula (K), Formula (V), and Formula (Z) compounds are preferably prepared by acid catalyzed hydrolysis of the corresponding N-($C_{1-3}$ alkyl)-($C_{1-3}$ alkanmamido) compounds as described above for the aminophenyl substituted compounds, or alternatively by reduction of the corresponding ($C_{1-3}$ alkanamido) phenyl Formula (K), Formula (V) or Formula (Z) compounds with borane or borane dimethylsulfide complex in tetrahydrofuran.

N,N-($C_{1-3}$ dialkylamino) phenyl substituted Formula (K) and Formula (Z) compounds are alternatively prepared by reduction of the corresponding N-($C_{1-3}$ alkyl)-($C_{1-3}$ alkanamido) compounds with borane as described above for the N-($C_{1-3}$ alkylamino) phenyl substituted compounds.

N-(azacyclo $C_{5-6}$ alkyl) phenyl substituted Formula (K) and Formula (Z) compounds are alternatively prepared by cyclodialkylation of the corresponding aminophenyl compounds with dibromobutane or dibromopentane and anhydrous potassium carbonate in an inert solvent such as dimethylformamide.

Compounds of Formula (Z) where X is $S(O)_n$ and n is 1 or 2 are prepared by oxidation with one or two equivalents of an organic peracid.

Compounds of Formula (Z) where at least one of $R^3$ and $R^2$ is pyridyl, X is $S(O)_n$ and n is 2 are preferably prepared by oxidation of 1 equivalent of an acid salt of a Formula (Z) compound where X is S-$(O)_n$,and n is 1, with 2/3 equivalent of an aqueous solution of potassium permanganate followed by dissolution of the resulting manganese dioxide with sulfur dioxide.

Compounds of Formula (Z) where X is $S(O)_n$, n is 1 or 2, and where at least one of $R^2$ and $R^3$ is $C_{1-3}$ alkylaminophenyl, $C_{1-3}$ dialkylaminophenyl, or N-(azacyclo $C_{5-6}$ alkyl)phenyl are preferably prepared by treating the immediate precursor alkanamidophenyl Formula (Z) compound with an oxidizing agent (as described above for preparation of compounds of Formula (ZA) where X is $S(O)_n$ and n is 1 or 2) followed by hydrolysis of the alkanamide to the primary or secondary amine. The primary or secondary amine may then be further alkylated as described above to afford the tertiary amine $S(O)_n$ compounds where n is 1 or 2.

The compounds of Formula (Z) can also be prepared according to the alternate synthetic route 2.

All the compounds of Formula (E), Formula (F), Formula (G) and Formula (H) are useful as intermediates in the preparation of compounds of Formula (Z) wherein X is $CH_2$. All of the necessary compounds of Formula (A), Formula (B) Formula (C) and Formula (D) can be obtained from commercial sources or are preparable by conventional techniques such as those set out herein.

Compounds of Formula (B), wherein B and C are H, methyl, ethyl, or gem-dimethyl, can be prepared by 0-alkylation of the corresponding 2-piperidone or 2-pyrrolidone of Formula (A), wherein B and C are as defined above, with dimethyl-sulfate. The necessary compounds of Formula (A) are commercially available or are prepared by known techniques. Compounds of Formula (C) wherein B and C are as defined above, can be prepared by treatment of the corresponding compound of Formula (B) with ammonium chloride in absolute ethanol. Compounds of Formula (C) wherein B and C are H are preferably prepared as their hydrohalide salts and liberated to the bases with concentrated aqueous sodium hydroxide or preferably with one equivalent of sodium $C_{1-2}$ alkoxide in the $C_{1-2}$ alcohol, followed by evaporation of the solvent in vacuo. Compounds of Formula (D), wherein $Y^3$ is Br and Y is the same as defined above in Formula (H), are commercially available or are prepared by treatment of the correspondingly substituted acetophenone in methylene chloride, chloroform or acetic acid with one equivalent of bromine, or alternatively, by reaction in chloroform-ethyl acetate with a suspension of copper (II) bromide. The necessary acetophenones are

commercially available or preparable by known techniques. Alternatively the Formula (D) compounds, wherein $Y^3$ is chloro and Y is (a) phenyl or 4-mono-substituted phenyl where the substituent is selected from halo, $C_{1-4}$ alkyl, $C_{1-3}$ alkylthio, $C_{1-3}$ alkoxy, or (b) 3,4-disubstituted phenyl wherein the substituents are the same and are selected from $C_{1-3}$ alkoxy, methylenedioxy, or where the substituents are independently selected from halo or $C_{1-3}$ alkoxy, can be prepared by acylating the corresponding mono- or disubstituted benzene by Friedel Crafts reaction with 2-chloroacetyl chloride and aluminium chloride.

Preferably, compounds of Formula (E) are prepared from their corresponding compound of Formula (H). Compounds of Formula (H) serve as intermediates in the preparation of compounds of Formula (E). Compounds of Formula (H) are prepared by treatment of a solution of a substituted Formula (D) compound, such as a 2-haloacetophenone, or a 2,3, or 4-bromoacetylpyridine, in a neutral, preferably nonpolar solvent with one molar equivalent of the corresponding Formula (C) compound, maintaining the temperature at or below 25°C. The resulting crystalline Formula (H) hydrohalide salts are converted to Formula (E) compounds by refluxing in water. Compounds of Formula (E) serve as intermediates in the preparation of the compounds of Formula (Z) when X is $CH_2$. Alternatively, compounds of Formula (E) are prepared by treatment of a solution of the 2-iminopyrrolidine or 2-iminopiperidine with a substituted 2-bromoacetophenone of Formula (D), either in a polar organic solvent, such as dimethylformamide or ethanol, or in a nonpolar chlorinated hydrocarbon, followed by removing all or most of the solvent and refluxing the residue in aqueous solution.

Compounds of Formula (Z) where X is $CH_2$, $r^3$ is phenyl or substituted phenyl, and $R^2$ is 4-pyridyl are preferably prepared in two steps. In the first step, the corresponding compound of Formula (E) is treated, preferably at 20-25°C, with an excess of both pyridine and an acyl halide or a haloacyl ester such as acetyl bromide, benzoylchloride, benzyl chloroformate, or preferably ethyl chloroformate, in an organic solvent in which the reactants are soluble and inert to form the compound of Formula (F). The acyl group can contain $C_{1-8}$ alkyl, $C_{1-8}$ alkoxy, phenyl, phenoxy, benzyl, or benzyloxy. One skilled in the art will recognize that the pyridine and acyl ester react to form the acylpyridinium reagent in situ. Optionally, the acylpyridinium reagent can be prepared separately in a solvent and then added to the solution of Formula (E) compound. Suitable solvents include methylene chloride, ethylene chloride, chloroform, carbon tetrachloride, tetrahydrofuran, ethyl ether, dioxane, toluene, or excess pyridine.

The reaction mixture is cooled as necessary by use of an ice/water bath during the mixing of the reactants, in order to maintain ambient temperature. The mixture is then allowed to stir at ambient temperature for up to 48 hours until reaction is complete. The reaction mixture is continually assayed using high pressure liquid chromatography (HPLC) or thin layer chromatography (TLC) on aliquots to ascertain if unreacted Formula (E) compound is present. If so, additional acyl pyridinium salt is introduced. Other conditions of the reaction are standard to the art. Following the reaction, the resultant compounds can be recovered from the reaction mixture and isolated by standard techniques. Compounds of Formula (F) serve as intermediates in the preparation of the compounds of Formula (F). In the second step, the Formula (F) compound, a dihydropyridine product, is deacylated and aromatized with sulfur in refluxing decalin, tetralin, p-cymene or xylene, or preferably with potassium tert-butoxide in tert-butanol with oxygen gas at reflux for 15 minutes to afford the corresponding compound of Formula (Z).

The same Formula (E) compounds used to prepare the 4-pyridyl Formula (Z) compounds where X is $CH_2$ are employed to prepare the analogous 2-pyridyl and 3-pyridyl Formula (Z) compounds. Treatment of one equivalent Formula (E) compound with one equivalent of bromine in methylene chloride at 20-25°C for about 1/2 hour, followed by addition of 5% potassium carbonate and concentration of the organic phase in vacuo results in 3-bromination to afford the 3-bromo-2-(substituted phenyl)-6,7-dihydro-(5H-pyrrolo[1,2-a]-imidazoles and 3-bromo-2-(substituted phenyl)-5,6,7,8-tetrahydro-imidazo[1,2-a]pyridines compounds of Formula (G). The compounds of Formula (G) serve as intermediates in the preparation of compounds of Formula (Z). These Formula (G) compounds are treated with n-butyl lithium (n-BuLi) in tetrahydrofuran to afford their 3-lithio derivatives by halogen-metal interchange.

Transmetallation of the 3-lithio compounds with $MgBr_2$ or $ZnCl_2$ to the corresponding magnesium or zinc compounds, provides good coupling to 2- or 3- bromopyridine in the presence of $PdCl_2$(1,4-bis-(diphenylphosphino)butane) catalyst, a bidentate Pd (II) catalyst. Alternatively the Formula (G) compounds may be coupled to the 2 or 3-metalated pyridine employing this bidentate Pd (II) catalyst, or the corresponding $Ni(II)Cl_2$-(1,2-bis(diphenylphosphino)ethane) catalyst. By either of these routes, Formula (Z) compounds are obtained where $R^2$ is 2-pyridyl or 3-pyridyl.

Compounds of Formula (F) can be prepared by addition of a Grignard reagent prepared from a Formula (G) compound to an N-acylpyridinium salt by the method previously described for the reaction of Grignard reagents prepared from Formula (V) compounds with N-acylpyridinium salt. The Grignard reagent is prepared from a compound of Formula (G) by the method previously described for the preparation of the

analogous Grignard reagent from a Formula (V) compound.

Regioisomers of Formula (Z) compounds where X is $CH_2$, $R^2$ is substituted phenyl, or 2-, 3-, or 4-pyridyl and $R^3$ is 2-, 3-, or 4-pyridyl are obtained from compounds of Formula (E) where $Y^4$ is 2-, 3-, or 4-pyridyl. Compounds of Formula (E) where $Y^4$ is 2-, 3-, or 4-pyridyl are prepared by treatment of a 2-, 3-, or 4-bromoacetylpyridine hydrobromide salt of Formula (D), wherein R is 2-, 3, or 4-pyridyl with 2-3 equivalents of the 2-iminopyrrolidine or 2-iminopiperidine by the procedure used to prepare the other compounds of Formula (E) described above. 3-Bromination affords the corresponding Formula (G) compounds. Metallation of the Formula (G) compounds via halogen-metal interchange with n-BuLi, transmetallation with $MgBr_2$ and coupling to the substituted bromobenzene or 2-, 3-, or 4-bromopyridine in the presence of the bidentate phosphine-palladium or nickel complex as described above affords the desired regioisomers of Formula (Z) and the bis(pyridyl) compounds of Formula (Z). Alternatively the metallated pyridine or substituted benzene may be coupled to the Formula (G) compounds employing the catalysts as described above.

Compounds of Formula (Z) where X is $CH_2$, $R^3$ or $R^2$ are $C_{1-3}$ alkylsulfinyl substituted phenyl are prepared by treatment of one equivalent of the corresponding compound of Formula (Z) where $R^3$ or $R^2$ are $C_{1-3}$ alkylmercaptophenyl, with one equivalent of an oxidizing agent (preferably, 3-chloroperbenzoic acid) per mercapto function, in an inert solvent. Compounds of Formula (Z) wherein X is $CH_2$, $R^3$ or $R^2$ are $C_{1-3}$ alkylsulfonyl substituted phenyl are prepared by treatment of one equivalent of the corresponding $C_{1-3}$ sulfinyl Formula (Z) with two-thirds equivalent of potassium permanganate per sulfinyl function in aqueous solution. After addition of all potassium permanganate, sulfur dioxide is passed through the solution to dissolve any precipitated manganese dioxide. The resulting liquor is extracted with chloroform, dried, evaporated, and the residue recrystallized from 50% aqueous alcohol to yield the desired product.

$C_{1-3}$ alkanamido and N-($C_{1-3}$ alkyl)-($C_{1-3}$ alkanamido)phenyl substituted acetophenones, and in some cases the Formula (E) and Formula (Z) compounds, are prepared by acylation of the corresponding amino and $C_{1-3}$ alkylamino compounds with the alkanoic acid anhydride or chloride in pyridine. Another alternative preparation of the N-($C_{1-3}$ alkyl)-($C_{1-3}$ alkanamido) phenyl substituted Formula (E) and Formula (Z) compounds is the alkylation of the corresponding $C_{1-3}$ alkan-amido substituted compounds with sodium hydride and a $C_{1-3}$ alkyl bromide or iodide in dimethyl formamide.

Aminophenyl substituted Formula (E) and Formula (Z) compounds are prepared either by hydrolysis of the corresponding $C_{1-3}$ alkanamido compounds in refluxing 6 N mineral acid or by catalytic reduction of the corresponding nitro compounds.

N-($C_{1-3}$ alkylamino)phenyl substituted Formula (E), Formula (G), and Formula (Z) compounds are preferably prepared by acid catalyzed hydrolysis of the corresponding N-($C_{1-3}$ alkyl)-$C_{1-3}$ alkanamido) compounds of Formula (E), Formula (G) and Formula (Z), respectively, prepared as described above for the aminophenyl substituted compounds, or alternatively, either by (a) reduction of the corresponding $C_{1-3}$ alkanamido compounds with borane or borane dimethylsulfide complex in tetrahydrofuran, or (b) by cleavage of the corresponding N,N-($C_{1-3}$ dialkylamino)phenyl substituted Formula (E) and Formula (Z) compounds with cyanogen bromide in the Von Braun reaction.

N,N-($C_{1-3}$ dialkylamino)phenyl substituted Formula (E) and Formula (Z) compounds are alternatively prepared either by reduction of the corresponding N-($C_{1-3}$ alkyl)-($C_{1-3}$ alkanamido) compounds of Formula (E) and Formula (Z) with borane as described above for the N-($C_{1-3}$ alkylamino)phenyl substituted compounds.

N-(azacyclo $C_{5-6}$ alkyl)phenyl substituted Formula (E) and Formula (Z) compounds are alternatively prepared by cyclodialkylation of the corresponding aminophenyl compounds with dibromobutane or dibromopentane and anhydrous potassium carbonate in an inert solvent such as dimethylformamide.

Compounds of Formula (E) where $Y^4$ is 2,2,2-trihaloethoxy or prop-2-ene-1-oxy substituted phenyl are prepared by alkylation of the appropriate phenols of Formula (E) with trifluoromethylsulfonic acid 2,2,2-trifluoroethyl ester or allyl bromide respectively as previously described for the Formula (K) compounds. Appropriately substituted mono and dihydroxy phenyl compounds of Formula (E) and Formula (Z) are obtained by treatment of their respective correspondingly substituted methoxy derivatives with HBr in acetic acid, or preferably with $BBr_3$ in methylene chloride at -60 °C followed by a return to room temperature, addition of water, and filtering of the crude product.

Pharmaceutically acceptable salts and their preparation are well known to those skilled in pharmaceuticals. Pharmaceutically acceptable salts of the compounds of Formulae (Z), (K), (J), and (V) which are useful in the present invention include maleate, fumarate, lactate, oxalate, methanesulfonate, ethanesulfonate, benzenesulfonate, tartrate, citrate, hydrochloride, hydrobromide, sulfate and phosphate salts. Preferred pharmaceutically acceptable salts of the compounds of Formula (Z) include hydrochloride and hydrobromide salts, and such salts can be prepared by known techniques.

By the term "inhibiting the production of IL-1" is meant the down regulation of excessive in vivo IL-1 levels in a human to normal levels.

By the term "production of IL-1 by monocytes and/or macrophages" is meant the in vivo release of IL-1 by such cells.

Interleukin-1 (IL-1) has been recently demonstrated to mediate a variety of biological activities thought to be important in immunoregulation and other physiological conditions such as inflammation [See, e.g., Dinarello et al., Rev. Infect. Disease, 6, 51 (1984)]. The myriad of known biological activities of IL-1 include the activation of T helper cells, induction of fever, stimulation of prostaglandin or collagenase production, neutrophil chemotaxis, induction of acute phase proteins and the suppression of plasma iron levels. However, much remains to be learned about the synthesis, processing and secretion of IL-1. For example, there is recent evidence suggesting that there are two separate human interleukin-1 genes, and that the products of these two genes differ in their isoelectric points. It is also clear that the published data on the cloning of the cDNA of the IL-1 gene(s) suggest that IL-1 is synthesized as a 31 kilodalton (Kd) precursor, which is subsequently processed to yield a smaller mature protein of about 17 Kd, the activity of which is detectable in culture supernatants. One interesting feature of the precursor protein is that it lacks a classical signal peptide sequence, suggesting that the molecule is probably not secreted in a classical manner. There is very little information available as to how the 31 Kd precursor is processed and secreted.

The discovery of a compound which specifically inhibits IL-1 production will not only contribute to the understanding of how this molecule is synthesized, processed and secreted, but will also provide a therapeutic approach for diseases in which excessive or unregulated IL-1 production is implicated.

It has now been discovered that compounds of Formula (I) and pharmaceutically acceptable salts thereof are useful for inhibiting the production of IL-1 by monocytes and/or macrophages in a human in need of such inhibition.

There are several disease states in which excessive or unregulated IL-1 production by monocytes and/or macrophages is implicated in exacerbating and/or causing the disease. These include rheumatoid arthritis [See, e.g., Fontana et al., Arthritis Rheum., 22, 49-53 (1982)]; osteoarthritis [See, e.g., Wood et al., Arthritis Rheum., 26, 975 (1983)]; toxic shock syndrome [See, e.g., Ikejima and Dinarello, J. Leukocyte Biology, 37, 714 (1985)]; other acute or chronic inflammatory disease states such as the inflammatory reaction induced by endotoxin [See, e.g., Habicht and Beck, J. Leukocyte Biology, 37, 709 (1985)]; and other chronic inflammatory disease states such as tuberculosis. [See, e.g., Chesque et al., J. Leukocyte Biology, 37, 690 (1985)]. Benjamin et al., "Annual Reports in Medicinal Chemistry - 20", Chapter 18, pages 173-183 (1985), Academic Press, Inc., disclose that excessive IL-1 production is implicated in: Psoriatic arthritis, Reiter's syndrome, Rheumatoid arthritis, Osteoarthritis, Gout, Traumatic arthritis, Rubella arthritis, and Acute synovitis.

Dinarello, J. Clinical Immunology, 5 (5), 287-297 (1985), reviews the biological activities which have been attributed to IL-1 and such activities are summarized in Table A.

Table A

Biological Activities Attributed to IL-1

- Fever (in rabbits, mice and rats)
- Hypoferremia
- Hypozincemia
- Hypercupremia
- Increased
  - Blood neutrophils
  - Hepatic acute-phase proteins
  - Bone resorption
  - Cartilage breakdown
  - Muscle proteolysis
  - Slow-wave sleep
  - Endothelial procoagulant
  - Chondrocyte proteases
  - Synovial collagenase
  - Endothelial neutrophil adherence
  - Neutrophil degranulation
  - Neutrophil superoxide
  - Interferon production
- Proliferation of
  - Fibroblasts
  - Glial cells
  - Mesangial cells
  - Synovial fibroblasts
  - EBV B-cell lines
- Chemotaxis of
  - Monocytes
  - Neutrophils
  - Lymphocytes

Table A

Biological Activities Attributed to IL-1

(Cont'd)

Stimulation of $PGE_2$ in
- Hypothalamus
- Cortex
- Skeletal muscle
- Dermal fibroblast
- Chondrocyte
- Macrophage/monocyte
- Endothelium ($PGI_2$)

Decreased
- Hepatic albumin synthesis
- Appetite
- Brain binding of opioids

Augmentation of
- T-cell responses
- B-cell responses
- NK activity
- IL-2 production
- Lymphokine production

An effective, IL-1 production inhibiting amount of a compound of Formula (I) or a pharmaceutically acceptable salt thereof is useful in treating, prophylactically or therapeutically, any disease state in a human which is exacerbated or caused by excessive or unregulated IL-1 production by such human's monocytes and/or macrophages. Preferably, the disease state is endotoxin-induced shock.

This invention relates to a method of inhibiting the production of IL-1 by monocytes and/or macrophages in a human in need thereof which comprises administering an effective, IL-1 production inhibiting amount of a compound of Formula (I) or a pharmaceutically acceptable salt thereof to such human. A compound of Formula (I) or a pharmaceutically acceptable salt thereof can be administered to such human in a conventional dosage form prepared by combining a compound of Formula (I), or a pharmaceutically acceptable salt thereof, with a conventional pharmaceutically acceptable carrier or diluent according to known techniques, such as those described in Bender et al., U.S.S.N. 856,875 filed April 28, 1986 and Bender et al., U.S.S.N. 856,928, filed April 28, 1986. It will be recognized by one of skill in the art that the form and character of the pharmaceutically acceptable carrier or diluent is dictated by the amount of active ingredient with which it is to be combined, the route of administration and other well-known variables. A compound of Formula (I) or a pharmaceutically acceptable salt thereof is administered to a human in need of inhibition of IL-1 production by its monocytes and/or macrophages in an amount sufficient to inhibit such excessive IL-1 production to the extent that it is regulated down to normal levels. The route of administration may be oral, parenteral or topical. The term parenteral as used herein includes intravenous, intramuscular, subcutaneous intranasal, intrarectal, intravaginal or intraperitoneal administration. The subcutaneous and intramuscular forms of parenteral administration are generally preferred. The daily oral dosage regimen will preferably be from about 5 to about 100 mg/kilogram of total body weight. The daily parenteral dosage regimen will preferably be from about 2 to about 80 mg per kilogram (kg) of total body weight, most preferably from about 3 to about 60 mg/kg. The daily topical dosage regimen will preferably be from about 2 mg to about 10 mg per site of administration. It will be recognized by one of skill in the art that the

optimal quantity and spacing of individual dosages of a compound of Formula (I) or a pharmaceutically acceptable salt thereof will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of a compound of Formula (I) or a pharmaceutically acceptable salt thereof given per day for a defined number of days, can be ascertained by those skilled in the art using conventional course of treatment determination tests.

## EXAMPLES

Without further elaboration, it is believed that one skilled in the art can, using the preceding description, utilize the present invention to its fullest extent. The following Examples are, therefore, to be construed as merely illustrative.

As used herein, the term "Compound 1" refers to the compound of Formula (I) wherein $R^1$ is 4-pyridyl, $R^2$ is 4-fluorophenyl, X is S(0)n and n is 0, and the term "Compound 5" refers to the compound of Formula (I) wherein $R^1$ is 4-pyridyl, $R^2$ is 4-flurophenyl and X is $CH_2$.

## EXAMPLE 1

Inhibitory Effect of a Compound of Formula (I) on in vitro IL-Production by Human Monocytes

The effects of antiinflammatory/antiarthritic drugs of different classes, including Compound 1 on the in vitro production of IL-1 by human monocytes was examined.

Bacterial lipopolysaccharide (LPS) was used to induce IL-1 production by human peripheral blood monocytes. IL-1 activity was measured by its ability to stimulate a Interleukin 2 (IL-2) producing cell line (EL-4) to secrete IL-2, in concert with A23187 ionophore, according to the method of Simon et al., J. Immunol. Methods, 84, 85, (1985).

Human peripheral blood monocytes were isolated and purified from either fresh blood preparations from volunteer donors, or from blood Bank buffy coats, according to the procedure of Colotta et al., J. Immunol., 132, 936 (1984). 1 X $10^6$ of such monocytes are plated in 24-well plates at a concentration of 2 million/ml per well. The cells were allowed to adhere for 2 hours, after which time non-adherent cells were removed by gentle washing. Test compounds were then added to the cells for 1 hour (hr) before the addition of lipopolysaccharide (10 ng/ml unless otherwise noted), and the cultures were incubated at 37°C for an additional 24 hours. At the end of the incubation period, culture supernatants were removed and clarified of cells and all debris. Culture supernatants were immediately assayed for IL-1 biological activity as well as prostaglandin and/or leukotriene concentrations by radioimmunoassay.

The results indicated that human peripheral blood monocytes are exquisitely sensitive to bacterial endotoxin. Nanogram or even picogram quantities of LPS stimulated high levels of IL-1 production as well as prostaglandin production; however, little, if any, leukotriene was detected in such supernatants. These observations are consistent with previous reports [(See, Humes et al., J. Biol. Chem., 257, 1591 (1982)]. As shown in Table 1, ibuprofen, although highly active in inhibiting prostaglandin synthesis, had virtually no effect on IL-1 production. The 5-lipoxygenase (5-LO) inhibitors phenidone and nordihydroguaiaretic acid (NDGA) were marginally active in inhibition of IL-1 production and, interestingly, had little inhibitory effect on prostaglandin synthesis. Compound 1, however, was active in submicromolar range ($10^{-7}$M), resulting in greater than 80% inhibition of both prostaglandin synthesis and IL-1 production. Chloroquine, an antiarthritic/antimalarial compound previously demonstrated to inhibit IL-1 production, [See, Lipsky, Amer. J. Med. 75, Supp. 1A, 19 (1983)] was only marginally active at doses one or two logs higher than that of Compound 1.

Inhibition of IL-1 production by Compound 1 is not due to a carryover effect of the drug on the cells used in the IL-1 readout assay. Various doses of Compound 1 were tested for its activity effects on a preparation of Il-1 standard on the EL-4/CTLL assay. At high concentrations (i.e., $10^{-5}$ or higher), compound 1 had an effect on the assay system, perhaps a result of toxic effects on the cells; however, at lower doses of Compound 1 there was no direct effect on the IL-1 assay per se. It has been further demonstrated that Compound 1 does not exert a nonspecific toxic effect on other functions of activated monocytes. LPS-treated human monocytes can mediate a potent tumoricidal activity in the kill of A375 melanoma cells, as previously demonstrated [See, Kleinerman et al., J. Clin. Inves., 72, 304 (1983)]. Compound 1, at concentrations ranging from $10^{-5}$ to $10^{-8}$M did not effect that biological activity. Furthermore, the possibility that Compound 1 treatment of monocytes in presence or absence of LPS may have induced an

inhibitor which interfered with the IL-1 assay was also ruled out (data not shown).

The in vitro inhibition of IL-1 production by Compound 1 is dose dependent. There is little, if any, difference between the effect of the drug at any given concentration of LPS. This observation, along with the fact that there was always a residual IL-1 activity remaining over a wide range of LPS used, suggests that the inhibitory effect of Compound 1 on IL-1 production remains constant, i.e., about 80% inhibition of $10^{-6}$ M and about 40% inhibition at $10^{-7}$ M.

The exact mechanism by which Compound 1 inhibits in vitro IL-1 production by monocytes is not presently known. It is clear, however, that while Compound 1 inhibits IL-1 production induced by a variety of activators, its inhibition is IL-1 specific, i.e., other inducible proteins such as alpha interferon are not effected. It is also clear that Compound 1 is not immunosuppressive and does not inhibit lectin-stimulated mitogenesis of human peripheral blood lymphocytes. Furthermore, it does not inhibit either IL-2 production or response.

The data in Table 1 shows that Compound 1 inhibits IL-1 production by human monocytes in vitro. This inhibitory activity does not seem to correlate with the property of the compound in mediating arachidonic acid metabolism inhibition since other nonsteroidal antiinflammatory drugs with potent cyclooxygenase and/or lipoxygenase inhibitory activity do not inhibit IL-1 production at nontoxic doses. Furthermore, inhibition of prostaglandin and/or leukotriene synthesis by a compound does not correlate with its ability to inhibit IL-1 production.

TABLE I

| Compound[a] | M | % Inhibition | |
|---|---|---|---|
| | | $PGE_2$ [c] | IL-1 |
| Ibuprofen | $10^{-5}$<br>$10^{-6}$<br>$10^{-7}$ | 97<br>71<br>63 | 9<br>0<br>0 |
| Phenidone | $10^{-7}$<br>$10^{-6}$<br>$10^{-7}$ | 63<br>0<br>0 | 0<br>18<br>0 |
| NDGA | $10^{-5}$[b]<br>$10^{-6}$<br>$10^{-7}$ | 25<br>0<br>0 | 88<br>33<br>31 |
| Compound 1 | $10^{-5}$<br>$10^{-6}$<br>$10^{-7}$ | 99<br>94<br>79 | 99<br>96<br>88 |
| Chloroquine | $10^{-5}$<br>$10^{-6}$<br>$10^{-7}$ | 84<br>0<br>0 | 89<br>46<br>36 |

(a) All compounds, except chloroquine, were dissolved in absolute ethanol at $10^{-2}$M, which was subsequently diluted further in tissue culture media. Ethanol control at dilution indicated that it did not affect the IL-1 assay. None of the compounds had a direct effect on the assay system at doses tested. Furthermore, test samples were dialyzed prior to testing in the IL-1 assay.
(b) At doses at or above $10^{-5}$ M, this compound exerted some toxic effects on monocytes, i.e., viability ~50% after 24 hours.
(c) $PGE_2$ was assayed using an RIA kit.

EXAMPLE 2

Inhibitory Effect of Major Metabolites of Compound 1 on In Vitro Production of IL-1 by Human Monocytes

In order to elucidate the mechanism by which Compound 1 mediates its anti-inflammatory activity, the ability of this compound and two closely related analog metabolites, (i.e., the analogs of Compound 1 wherein X is S(0)n and n is 1 or 2) to inhibit human IL-1 production in vitro was evaluated according to the method of Example 1. Briefly, 1 x $10^6$ human peripheral blood monocytes were plated in 24-well plates and allowed to adhere for 1 hr at 37 °C. The above compounds were added to a final concentration of $10^{-5}$ M to $10^{-8}$ M. The monocytes were stimulated to produce IL-1 with 1 ng/ml LPS after a 1 hr pretreatment of the cells with the respective compounds. Table 2 summarizes the results obtained and shows that all three compounds of Formula (I) dramatically inhibit IL-1 production. Compound 1 appears to be slightly more potent than the two analogs tested.

Table 2

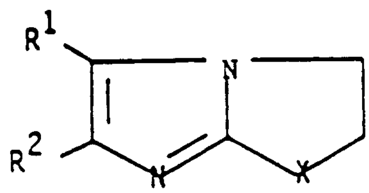

Formula (I)

| Compound No. | $R^1$ | $R^2$ | X | n | $IC_{50}$ (µM) |
|---|---|---|---|---|---|
| 1 | 4-pyridyl | 4-fluorophenyl | S(0)n | 0 | 0.25 |
| 2 | 4-pyridyl | 4-fluorophenyl | S(0)n | 1 | 0.80 |
| 3 | 4-pyridyl | 4-fluorophenyl | S(0)n | 2 | 0.61 |

EXAMPLE 3

Effect of Compounds of Formula (I) on IL-1 Production by Human Monocytes

The inhibitory effect of additional compounds of Formula (I) on the in vitro production of IL-1 production of IL-1 by human monocytes was determined according to the procedure of Examples 1 and 2. Table 3 summarizes the results obtained. As indicated by Table 3, all compounds of Formula (I) inhibit IL-1 production.

## TABLE 3

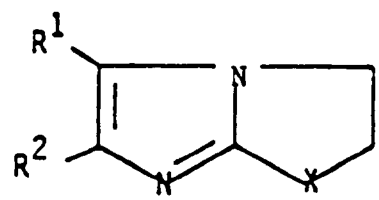

Formula I

| Compound No. | $R_1$ | $R_2$ | X | n | % Inhibition @ $10^{-6}M$ |
|---|---|---|---|---|---|
| 1 | 4-pyridyl | Fϕ a) | S(O)n | 0 | 70 |
| 2 | 4-pyridyl | Fϕ | S(O)n | 1 | 56 |
| 3 | 4-pyridyl | Fϕ | S(O)n | 2 | 54 |
| 4 | Fϕ | 4-pyridyl | S(O)n | 0 | 46 |
| 5 | 4-pyridyl | Fϕ | $CH_2$ | - | 78 |
| 6 | 4-pyridyl | MeOϕ (b) | S(O)n | 0 | 64 |
| 7 | 4-pyridyl | MeOϕ | $CH_2$ | - | 40 |
| 8 | 4-pyridyl | MeOϕ | $CH_2CH_2$ | - | 30 |

(a)Fϕ = 4-fluorophenyl

(b)MeOϕ = 4-methoxyphenyl

EXAMPLE 4

Adjuvant-induced arthritis in the rat is a model of systemic inflammation with characteristics similar to rheumatoid arthritis in man. Arthritic rats exhibit many alterations associated with macrophage activation including increased production of IL-1 by LPS-stimulated splenic adherent macrophages in vitro. This model was employed to adherent macrophages in vitro. This model was employed to assess the effect of Compound 1 in vivo on this elevated IL-1 production. Briefly, rats were injected on day 0 with Freund's complete adjuvant in the right hind leg. Drugs were prepared as a suspension in tragacanth and administered daily per os either prophylactically (days 1 to 16) or therapeutically in a short-term modality (days 14 to 16). In all cases, final administration of drug occurred at 3 hours prior to sacrifice. Spleens were removed from these rats and the adherent cells were assessed for their ability to produce IL-1 in vitro in response to stimulation by LPS.

Tables 4a and 4b summarize the data from a typical experiment. In the case of prophylactic administration, Compound 1 produced a more dramatic decrease in IL-1 production than indomethacin which resulted in nearly complete normalization of this parameter. It is interesting to note that no effect was also observed in adjuvant arthritic rats therapeutically treated with Compound 1 or with a variety of clinically

effective and commercially marketed human antiarthritic agents such as auranofin, methotrexate, d-penicillamine and indomethacin. Hence, the prescribed short-term treatment of an ongoing arthritic disease in the adjuvant-induced arthritic rat model does not adequately reflect therapeutic efficacy of known or potentially useful antiarthritic drugs.

Based on the widely held belief of the role of unmodulated (i.e., excessive) in vivo IL-1 production in causing or aggravating inflammatory responses and other disease states (see, e.g., Fontana et al., supra; Wood et al., supra; Akejima and Dinarello, supra; Habicht and Beck, supra; Chesque et al., supra; Benjamin et al., supra; and Dinarello, supra), and based on the fact that compounds of Formula (I) inhibit in vitro IL-1 production by human macrophages and/or monocytes (see, Tables 1, 2 and 3), as well as the fact that a compound of Formula (I) prophylactically inhibited such IL-1 production in the adjuvant-induced arthritic rat (see, Table 4a), and also the fact that compounds of Formula (I) prophylactically inhibited LPS-induced endotoxin shock in a murine model (Example 5, infra), it is expected that all compounds of Formula (I) inhibit the in vivo IL-1 production by monocytes and/or macrophages in a human in need thereof when used according to the method of the subject invention.

TABLE 4a

% NORMAL CONTROL

PROPHYLACTIC STUDIES

| | AA | COMPOUND 1 | INDOMETHACIN | PREDNISOLONE | METHOTREXATE | D-PENICILLAMINE |
|---|---|---|---|---|---|---|
| RIGHT LEG PAW EDEMA | 197 ± 9 | 134 ± 9*** | 113 ± 8*** | 125 ± 22*** | 96 ± 4*** | 194 ± 20 |
| SPLENIC ADHERENT CELLS | | | | | | |
| % Adherent Cells | 357 ± 235 | 98 ± 29** | 480 ± 189 | 184 ± 79 | 134 ± 10* | 372 ± 117 |
| IL-1 Production | 368 ± 148 | 139 ± 28*** | 274 ± 100 | 168 ± 40** | 67 ± 56*** | 336 ± 170 |

PROPHYLACTIC TREATMENT: Drugs were administered daily p.o. as a suspension in tragacanth from day 1 to day 16. Final dose given 3 hrs before sacrifice.

| | | | |
|---|---|---|---|
| *** | $p < 0.01$ | Indomethacin | 2.0 mg/kg |
| ** | $p < 0.05$ | Compound 1 | 60 mg/kg |
| * | $p < 0.10$ | Prednisolone | 10 mg/kg |
| | | Methotrexate | 0.3 mg/kg |
| | | D-Penicillamine | 50 mg/kg |

TABLE 4b

% NORMAL CONTROL

THERAPEUTIC STUDIES

| | AA | COMPOUND I | INDOMETHACIN | PREDNISOLONE | METHOTREXATE | D-PENICILLAMINE |
|---|---|---|---|---|---|---|
| RIGHT LEG PAW EDEMA | 193 ± 6 | 159 ± 7*** | 145 ± 5*** | 147 ± 18*** | 194 ± 2 | 191 ± 13 |
| SPLENIC ADHERENT CELLS | | | | | | |
| % Adherent Cells | 271 ± 118 | 216 ± 137 | 394 ± 253 | 82 ± 47 | 284 ± 175 | 217 ± 6 |
| IL-1 Production | 461 ± 201 | 392 ± 129 | 330 ± 121 | 300 ± 280 | 436 ± 178 | 374 ± 187[a] |

THERAPEUTIC TREATMENT: Drugs were administered daily p.o. as above from day 14 to day 16. Final dose given 3 hrs before sacrifice.

*** $p < 0.01$

| | |
|---|---|
| Indomethacin | 2.0 mg/kg |
| Compound I | 60 mg/kg |
| Prednisolone | 10 mg/kg |
| Methotrexate | 0.3 mg/kg |
| D-Penicillamine | 50 mg/kg |

[a] N = 2

EXAMPLE 5

EFFECT OF COMPOUNDS OF FORMULA I IN ENDOTOXIN SHOCK

Model of endotoxin shock in C57B1/6 Mice

Mice are very resistant to the lethal effects of bacterial lipopolysaccharides (LPS). D-Galactosamine (D-GALN) induces a high degree of sensitization to the lethal effects of LPS in mice and other experimental animals. [See, Galanos et al., Proc. Natl. Acad Sci. USA., 76, 5939 (1979)]. In mice, depending on the strain and age, the sensitivity to endotoxin can be increased many thousand-fold.

A number of preliminary experiments were carried out to establish this model of lethal endotoxin shock. LPS concentrations of up to 1 mg/mouse are not lethal in C57B1/6 mice. When injected i.v. with D-GALN (500 mg/kg), however, the sensitivity increases so that 0.1 µg becomes a lethal dose (Table 5). The concentration of D-GALN required to obtain this effect is 400-500 mg/kg.

RESULTS

Dexamethasone provided protection when administered to the animals 24 hours and 1 hour prior to LPS/GALN administration.

Compound 1 also protected the animals from the lethal effects of LPS/GALN. Protection was obtained when Compound 1 was administerrd 1 hour prior to the injection of LPS/GALN and varied from 50 to 100% protection. Other compounds tested that have shown protective effects in this model are, Compound 5 phenidone (a dual inhibitor of arachidonic acid metabolism), and the histamine $H_1$ antagonist chlorpheniramine (but not the $H_2$ antagonist cimetidine).

Table A

| Lethality of LPS in D-Galactosamine Treated C57B1/6Mice | | | |
|---|---|---|---|
| Treatment | | Lethality No. of Death/Total No. | |
| | | 6 Hr. | 24 Hr. |
| LPS 100 µg | | 0/10 | 0/10 |
| D-Galactosamine | 500 mg/kg | 0/10 | 0/10 |
| D-Galactosamine | 500 mg/kg | | |
| + LPS | 1 µg | 8/10 | 10/10 |
| | .1 µg | 9/10 | 10/10 |
| | .01 µg | 1/10 | 5/10 |
| All compounds were administered i.v. | | | |

D-Galactosamine sensitized mice to the lethal effect of 0.1 µg of LPS. Treatment with .01 µg of LPS resulted in 50% lethality 6-24 hours following the injection of LPS/D-GALN.

The following EXAMPLES describe the synthesis of some of the compounds of Formula (1):

The following examples are illustrative of the invention. Temperatures are on the Centigrade scale.

EXAMPLE 1

Preparation of the 2-Thione Starting Material

To an aqueous solution (75 ml) of sodium cyanide (19.6 g; 400 mm) and benzyltriethyl ammonium chloride (3.0 g, 13 mm) was added isonicotinaldehyde (10 g, 93 mm) in 100 ml of methylene chloride at 0° temperature. Vigorous stirring was maintained for 15 minutes and a methylene chloride solution of benzoyl chloride (14.0 g, 100 mm, 50 ml) was slowly added. Cooling was discontinued after one-half hour reaction and the mixture was allowed to reach ambient temperature. The organic layer was separated and washed with 5% sodium carbonate and brine, dried and evaporated to an oil which was thoroughly extracted with ether (4 l). The ethereal solution was concentrated to approximately 500 ml and allowed to crystallize to give 5.0 g of isonicotinalde-hyde-o-benzoylcyanohydrin. The cyanohydrin (3.0 g, 12 mm) was stirred in 50

ml of tert.-butyl alcohol with the substituted benzaldehyde (12 mm) and sodium hydride (12 mm) was added. Stirring continued for 1-1/2 hours at room temperature and a mineral oil suspension of potassium hydride (24% suspension, 4 ml, 23 mm) was added with caution. A thin layer chromatographic assay of an aliquot of the reaction mixture after 1-1/2 hours indicated the formation of a single product. This was worked up by quenching the suspension in 200 ml of ice-water mixture and extracting with chloroform. The chloroform extract was evaporated to a crystalline residue which was further crystallized by the addition of ether. A crystalline product was obtained which could be further purified by crystallization from methylene chloride-ether mixtures.

2-(4-methylthiophenyl)-2-hydroxy-1-(4-pyridyl)ethanone; (60%) m.p. 127-130°.

| | | | |
|---|---|---|---|
| Anal. Calcd. 1/4 $H_2O$: | C, 63.73; | H, 5.15; | N, 5.31 |
| Found: | C, 63,33; | H, 5.12,; | N, 5.31 |

2-(4-methoxyphenyl)-2-hydroxy-1-(4-pyridyl)ethanone; (50%).

| | | | |
|---|---|---|---|
| Anal. Calcd. 1 $H_2O$: | C, 68.55; | H, 6.16; | N, 5.70 |
| Found: | C, 68.29; | H, 5.31; | N, 5.65 |

The above procedure was used with p-fluorobenzaldehyde but the intermediate was not isolated but was used directly in the next step.

9.0 G. (37 mm) of the hydroxy ketone was refluxed in 140 ml of dimethylformamide with 5.3 g (70 mm) of thiourea. Starting material was no longer detected after 4 hours of reaction and the solvent was concentrated to one half of the original volume to initiate the precipitation of the crystalline 2-mercaptoimidazole; crystallization was completed overnight in the refrigerator. The compounds were purified by trituration or crystallization from ethanol.

4-(4-Methoxyphenyl)-5-(4-pyridyl)-2-mercaptoimidazole was formed in 68% yield; m.p. 280°.

4-(4-Methylthiophenyl)-5-(4-pyridyl)-2-mercaptoimidazole was formed in 65% yield; m.p. 350°.

| | | | |
|---|---|---|---|
| Anal. Calcd.: | C, 60.17; | H, 4.38; | N, 14.03 |
| Found: | C, 60.11; | H, 4.71; | N, 14.25 |

4-(4-Fluorophenyl)-5-(4-pyridyl)-2-mercaptoimidazole was formed in 40% overall yield of crude material based on the benzoyl cyanonydrin condensation, m.p. 386-388°.

| | | | |
|---|---|---|---|
| Anal. Calcd. 1/8 $H_2O$: | C, 61.46; | H, 3.78; | N, 15.39 |
| Found: | C, 61.58; | H, 4.21; | N, 15.11 |

EXAMPLE 2

Formation of the Isomeric 5/6-Pyridyl-6/5-Phenyl-2,3-Dihydroimidazo[2,1-b]Thiazoles

The mercoptoimidazole (12.5 mm) was suspended in 100 ml of dimethylformamide and sodium hydride (13.0 mm) was added. Salt formation was allowed to proceed at room temperature for 1/2 hour at which time 1-bromo-2-chloroethane was added from a syringe and the solution was stirred overnight under an argon atmosphere.

Solid anhydrous potassium carbonate (20.0 mm) was added to the reaction mixture and reflux was initiated for 2-1/2 hours. Dilution of the dimethylformamide solution with ice-water mixture to 300 ml, caused precipitation of the organic products which were purified as described below.

EXAMPLE 3

Purification and Separation of 6-(4-Methoxyphenyl)-5-(4-Pyridyl)-2,3-Dihydroimidazo[2,1-b]Thiazole and 5-(4-Methoxyphenyl)-6-(4-Pyridyl)-2,3-Dihydroimidazo[2,1-b]Thiazole

The oily organic residue from the procedure of Example 2 was triturated with isopropanol and crystallized. The crystalline material that separated out was almost pure Va, ($R_f$:0.2 silica/ether). This preferred 6-(4-methoxyphenyl)-5-(4-pyridyl)isomer was recrystallized from chloroform-ether, m.p. 170-172°; 1.5 g.

| | | | |
|---|---|---|---|
| Anal. Calcd.: | C, 66.00; | H, 4.89; | N, 13.58 |
| Found: | C, 65.74; | H, 4.98; | N, 13.86 |

The mother liquor of the crystallization deposited a second crop of crystalline material, mainly Vb ($R_f$: 0.3, silica/ether). The mother liquor of this 2nd crop was then chromatographed on silica using the dry column technique with ethyl acetate. The faster moving substance was eluted, combined with the second crop of the isopropanol crystallization and both crystallized from chloroform-ether yielding 0.7 g, m.p. 187-188°, of the 5-(4-methoxyphenyl)-6-(4-pyridyl)isomer.

| | | | |
|---|---|---|---|
| Anal. Calcd. 1/4 $H_2O$: | C, 65.05; | H, 4.98; | N, 13.39 |
| Found: | C, 65.34; | H, 4.89; | N, 13.58 |

EXAMPLE 4

Purification and Separation of 6-(4-Methylthiophenyl)-5-(4 Pyridyl)-2,3-Dihydroimidazo[2,1-b]Thiazole and 5-(4-Methylthiophenyl)-6-(4-Pyridyl)-2,3-Dihydroimidazo[2,1-b]Thiazole

The oily residue from the procedure of Example 2 was extracted into ether (2 x 150 ml) and chloroform (2 x 100 ml). The ethereal extract deposited 1.5 g of a crystalline material, the chloroform extract was evaporated and taken up in isopropanol, ether was added (40 ml) to establish a 1:1 ratio and allowed to crystallize. The yield of this second action was 1.00 g. Both crystalline fractions were mixtures of the two 5,6-isomers which were separated by chromatography using silica with 20% isopropanol-ether solvent. The faster moving spot ($R_f$: .60, silica/ether- isopropanol) was first eluted and was further purified by crystallization from ether-chloroform, m.p. 210-213°, 0.6 g of the 5-(4-methylthiophenyl-6-(4-pyridyl)isomer.

| | | | |
|---|---|---|---|
| Anal. Calcd. 1/4 $H_2O$: | C, 61.88; | H, 4.73; | N, 12.73 |
| Found: | C, 62.16; | H, 4.93; | N, 12.57 |

The slower moving spot consisted mainly of the desired 6-(4-methylthiophenyl)-5-(4-pyridyl)isomer ($R_f$: 0.22) and was recrystallized from chloroform-ether (1:1), m.p. 190-193°, yield: 0.4 g.

| | | | |
|---|---|---|---|
| Anal. Calcd. 1/4 $H_2O$: | C, 61.88; | H, 4.73; | N, 12.73 |
| Found: | C, 61.99; | H, 4.87; | N, 12.54 |

## Claims

1. The use of a compound of the formula (I)

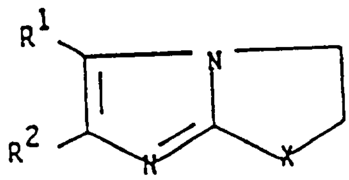

Formula (I)

wherein:

One of $R^1$ and $R^2$ must be 4-pyridyl and the other is selected from monosubstituted phenyl wherein said substituent is selected from halo or $C_{1-4}$ alkoxy;

X is $CH_2$, $CH_2CH_2$ or S(0)n; and

n is 0, 1 or 2;

or a pharmaceutically acceptable salt thereof; for the preparation of a medicament for inhibiting the production of interleukin-1 by monocytes and/or macrophages in a human suffering from a disease state in which excessive or unregulated IL-1 production by monocytes and/or macrophages is implicated in exacerbating and/or causing.

2. The use according to Claim 1 wherein the form of the medicament is suitable for parenteral administration.

3. The use according to Claim 2 wherein the form is suitable for intravenous or intramuscular administration.

4. The use according to Claim 1 wherein the form of the medicament is suitable for oral administration.

5. The use according to Claim 1 wherein the form of the medicament is suitable for topical administration.

6. The use of a compound of Claim 1 wherein $R^1$ is 4-pyridyl, $R^2$ is 4-fluorophenyl, X is S(0)n and n is 0.

7. The use of a compound of Claim 1 wherein $R^1$ is 4-pyridyl, $R^2$ is 4-fluorophenyl, X is S(0)n and n is 1.

8. The use of a compound of Claim 1 wherein $R^1$ is 4-pyridyl, $R^2$ is 4-fluorophenyl, X is S(0)n and n is 2.

9. The use of a compound of Claim 1 wherein $R^1$ is 4-fluorophenyl, $R^2$ is 4-pyridyl, X is S(0)n and n is 0.

10. The use of a compound of Claim 1 wherein $R^1$ is 4-pyridyl, $R^2$ is 4-fluorophenyl and X is $CH_2$.

11. The use of a compound of Claim 1 wherein $R^1$ is 4-pyridyl, $R^2$ is 4-methoxyphenyl, X is S(0)n and n is 0.

12. The use of a compound of Claim 1 wherein $R^1$ is 4-pyridyl, $R^2$ is 4-methoxyphenyl and X is $CH_2$.

13. The use of a compound of Claim 1 wherein, $R^1$ is 4-pyridyl, $R^2$ is 4-methoxyphenyl and X is $CH_2CH_2$.

14. The use according to Claim 1 wherein the human is afflicted with endotoxin-induced shock.

15. The use of a compound of Claim 14 wherein $R^1$ is 4-pyridyl, $R^2$ is 4-fluorophenyl, X is S(0)n and n is O.

16. The use of a compound of Claim 14 wherein $R^1$ is 4-pyridyl, $R^2$ is 4-fluorophenyl and X is $CH_2$.

## Patentansprüche

**1.** Verwendung einer Verbindung der Formel (I)

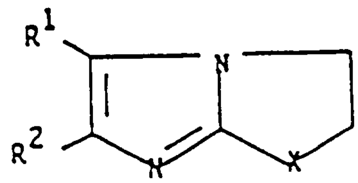

Formel (I)

in der:

einer der Reste $R^1$ und $R^2$ eine 4-Pyridylgruppe sein muß, und der andere aus einer monosubstituierten Phenylgruppe ausgewählt ist, wobei der Substituent aus einem Halogenatom oder einem $C_{1-4}$-Alkoxyrest ausgewählt ist;

X eine $CH_2$-, $CH_2CH_2$-Gruppe oder S(O)n bedeutet; und

n 0, 1 oder 2 ist;

oder ein pharmazeutisch verträgliches Salz davon; zur Herstellung eines Arzneimittels zur Inhibierung der durch Monozyten und/oder Makrophagen verursachten Bildung von Interleukin-1 in einem Menschen, der an einem Krankheitszustand leidet, bei dem eine durch Monozyten und/oder Makrophagen verursachte übermäßige oder unkontrollierte IL-1-Bildung an dessen Verschlimmerung und/oder verursachung beteiligt ist.

**2.** Verwendung nach Anspruch 1, wobei die Form des Arzneimittels für eine parenterale Verabreichung geeignet ist.

**3.** Verwendung nach Anspruch 2, wobei die Form für eine intravenöse oder intramuskuläre Verabreichung geeignet ist.

**4.** Verwendung nach Anspruch 1, wobei die Form des Arzneimittels für eine orale Verabreichung geeignet ist.

**5.** Verwendung nach Anspruch 1, wobei die Form des Arzneimittels für eine lokale Verabreichung geeignet ist.

**6.** Verwendung einer Verbindung nach Anspruch 1, wobei $R^1$ eine 4-Pyridylgruppe bedeutet, $R^2$ eine 4-Fluorphenylgruppe ist, X S(O)n bedeutet, und n 0 ist.

**7.** Verwendung einer Verbindung nach Anspruch 1, wobei $R^1$ eine 4-Pyridylgruppe bedeutet, $R^2$ eine 4-Fluorphenylgruppe ist, X S(O)n bedeutet, und n 1 ist.

**8.** Verwendung einer Verbindung nach Anspruch 1, wobei $R^1$ eine 4-Pyridylgruppe bedeutet, $R^2$ eine 4-Fluorphenylgruppe ist, X S(O)n bedeutet, und n 2 ist.

**9.** Verwendung einer Verbindung nach Anspruch 1, wobei $R^1$ eine 4-Fluorphenylgruppe bedeutet, $R^2$ eine 4-Pyridylgruppe ist, X S(O)n bedeutet, und n 0 ist.

**10.** Verwendung einer Verbindung nach Anspruch 1, wobei $R^1$ eine 4-Pyridylgruppe bedeutet, $R^2$ eine 4-Fluorphenylgruppe ist, und X eine $CH_2$-Gruppe bedeutet.

**11.** Verwendung einer Verbindung nach Anspruch 1, wobei $R^1$ eine 4-Pyridylgruppe bedeutet, $R^2$ eine 4-Methoxyphenylgruppe ist, X S(O)n bedeutet, und n 0 ist.

12. Verwendung einer Verbindung nach Anspruch 1, wobei $R^1$ eine 4-Pyridylgruppe bedeutet, $R^2$ eine 4-Methoxyphenylgruppe ist, und X eine $CH_2$-Gruppe bedeutet.

13. Verwendung einer Verbindung nach Anspruch 1, wobei $R^1$ eine 4-Pyridylgruppe bedeutet, $R^2$ eine 4-Methoxyphenylgruppe ist, und X eine $CH_2CH_2$-Gruppe bedeutet.

14. Verwendung nach Anspruch 1, bei einem Menschen, der an einem durch Endotoxin ausgelösten Schock leidet.

15. Verwendung einer Verbindung nach Anspruch 14, wobei $R^1$ eine 4-Pyridylgruppe bedeutet, $R^2$ eine 4-Fluorphenylgruppe ist, X S(O)n bedeutet, und n 0 ist.

16. Verwendung einer Verbindung nach Anspruch 14, wobei $R^1$ eine 4-Pyridylgruppe bedeutet, $R^2$ eine 4-Fluorphenylgruppe ist, und X eine $CH_2$-Gruppe bedeutet.

**Revendications**

1. Utilisation d'un composé de formule (I) :

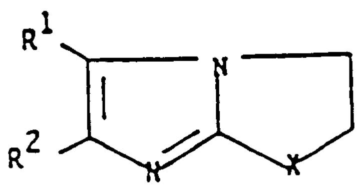

(I)

dans laquelle :

l'un des $R^1$ et $R^2$ doit être un groupe 4-pyridyle et l'autre est choisi parmi un groupe phényle monosubstitué dans lequel ce substituant est choisi parmi un atome d'halogène ou un groupe alcoxy en $C_{1-4}$;

X est $CH_2$, $CH_2CH_2$ ou $S(O)_n$; et

n est 0, 1 ou 2;

ou d'un sel de celui acceptable du point de vue pharmaceutique; pour la préparation d'un médicament pour inhiber la production d'interleukine-1 par des monocytes et/ou des macrophages chez un homme souffrant d'un état de maladie dans lequel une production excessive ou irrégulée de IL-1 par des monocytes et/ou des macrophages est impliquée en ce qu'elle exacerbe ou provoque celui-ci.

2. Utilisation suivant la revendication 1, dans laquelle la forme du médicament convient pour l'administration parentérale.

3. Utilisation suivant la revendication 2, dans laquelle la forme du médicament convient pour l'administration intraveineuse ou intramusculaire.

4. Utilisation suivant la revendication 1, dans laquelle la forme du médicament convient pour l'administration orale.

5. Utilisation suivant la revendication 1, dans laquelle la forme du médicament convient pour l'administration topique.

6. Utilisation d'un composé de la revendication 1, dans lequel $R^1$ est un groupe 4-pyridyle, $R^2$ est un groupe 4-fluorophényle, X est $S(O)_n$ et n est 0.

7. Utilisation d'un composé de la revendication 1, dans lequel $R^1$ est un groupe 4-pyridyle, $R^2$ est un groupe 4-fluorophényle, X est $S(O)_n$ et n est 1.

8. Utilisation d'un composé de la revendication 1, dans lequel $R^1$ est un groupe 4-pyridyle, $R^2$ est un groupe 4-fluorophényle, X est $S(O)_n$ et n est 2.

**9.** Utilisation d'un composé de la revendication 1, dans lequel $R^1$ est un groupe 4-fluorophényle, $R^2$ est un groupe 4-pyridyle, X est $S(O)_n$ et n est 0.

**10.** Utilisation d'un composé de la revendication 1, dans lequel $R^1$ est un groupe 4-pyridyle, $R^2$ est un groupe 4-fluorophényle, X est $CH_2$.

**11.** Utilisation d'un composé de la revendication 1, dans lequel $R^1$ est un groupe 4-pyridyle, $R^2$ est un groupe 4-méthoxyphényle, X est $S(O)_n$ et n est 0.

**12.** Utilisation d'un composé de la revendication 1, dans lequel $R^1$ est un groupe 4-pyridyle, $R^2$ est un groupe 4-méthoxyphényle, X est $CH_2$.

**13.** Utilisation d'un composé de la revendication 1, dans lequel $R^1$ est un groupe 4-pyridyle, $R^2$ est un groupe 4-méthoxyphényle, X est $CH_2CH_2$.

**14.** Utilisation suivant la revendication 1, dans laquelle l'homme est affligé d'un choc induit par une endotoxine.

**15.** Utilisation d'un composé de la revendication 14, dans lequel $R^1$ est un groupe 4-pyridyle, $R^2$ est un groupe 4-fluorophényle, X est $S(O)_n$ et n est 0.

**16.** Utilisation d'un composé de la revendication 14, dans lequel $R^1$ est un groupe 4-pyridyle, $R^2$ est un groupe 4-fluorophényle, X est $CH_2$.